# EUROPÄISCHE PATENTSCHRIFT

(11) **EP 1 453 476 B1**
(45) Veröffentlichungstag und Bekanntmachung des Hinweises auf die Patenterteilung: **03.05.2006**
(21) Anmeldenummer: 02791753.3
(22) Anmeldetag: 02.12.2002
(51) Int. Cl.: A61K 8/41, A61Q 5/10

(54) **NEUE OXIDATIONSFARBSTOFFE MIT 2,4-DICHLOR-3-AMINOPHENOL**
NOVEL OXIDATION DYES COMPRISING 2,4-DICHLORO-3-AMINOPHENOL
NOUVEAUX COLORANTS D'OXYDATION CONTENANT DU 2,4-DICHLORO-3-AMINOPHENOL

(30) Priorität: 11.12.2001 DE 10160815
(43) Veröffentlichungstag der Anmeldung: 08.09.2004
(73) Patentinhaber: Henkel Kommanditgesellschaft auf Aktien, 40589 Düsseldorf (DE)
(72) Erfinder: ROSE, David, 40723 Hilden (DE); HÖFFKES, Horst, 40595 Düsseldorf (DE)
(86) Internationale Anmeldenummer: PCT/EP2002/013588
(87) Internationale Veröffentlichungsnummer: WO 2003/053385

(56) Entgegenhaltungen:
- DE-A- 4 102 907
- DE-U- 20 017 642
- US-A- 4 129 414
- US-A- 4 629 466

## Beschreibung

Die vorliegende Erfindung betrifft Mittel zum Färben von Keratinfasern, die 2,4-Dichlor-3-aminophenol als Kupplerkomponente in Verbindung mit speziellen zweikernigen Entwicklerkomponenten enthalten, ein Verfahren zum Färben von Keratinfasem mit diesen Mitteln sowie die Verwendung dieser Farbstoffkombinationen zur Färbung von Keratinfasern.

Keratinfasern, insbesondere das menschliche Haar werden heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel empfindlich, so daß eine vielfach unerwünschte Nuancenverschiebung oder gar eine sichtbare "Entfärbung" eintreten kann.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kuppler-komponenten die eigentlichen Farbstoffe aus. Die Oxidationsfärbemittel zeichnen sich durch hervorragende, lang anhaltende Färbeergebnisse aus.

Gute Oxidationsfarbstoffvorprodukte sollen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Sie sollen beständig sein gegen Licht, Wärme, Schweiß, Reibung und den Einfluß chemischer Reduktionsmittel, z.B. Dauerwellenflüssigkeiten. Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein. Weiterhin soll die erzielte Färbung durch Blondierung leicht wieder aus dem Haar entfernt werden können, falls sie dennoch nicht den individuellen Wünschen der einzelnen Person entspricht und rückgängig gemacht werden soll.

Allein mit einer Entwicklerkomponente oder einer speziellen Kuppler/Entwicklerkombination gelingt es in der Regel nicht, eine auf dem Haar natürlich wirkende Farbnuance zu erhalten. In der Praxis werden daher üblicherweise Kombinationen verschiedener Entwickler- und/oder Kupplerkomponenten eingesetzt. Es besteht daher ständig Bedarf an neuen, verbesserten Farbstoffkomponenten, insbesondere um eine möglichst breite Abdeckung der gesamten Farbpalette zu erzielen.

Es wurde nunmehr überraschenderweise gefunden, daß besonders intensive Färbungen mit ausgezeichneten Echtheitseigenschaften und außerordentlich guten toxikologischen Eigenschaften erhalten werden können, wenn Färbemittel eingesetzt werden, die eine spezielle Kombination aus 2,4-Dichlor-3-aminophenol und mindestens einer zweikemigen Entwicklerkomponente enthalten.

Ein erster Gegenstand der vorliegenden Erfindung ist daher ein Mittel zum Färben von Keratinfasern, das in einem zum Färben geeigneten Medium als Oxidationsfarbstoffvorprodukte eine Kombination aus
a) 2,4-Dichlor-3-aminophenol und
b) mindestens einer Verbindung gemäß Formel (I) 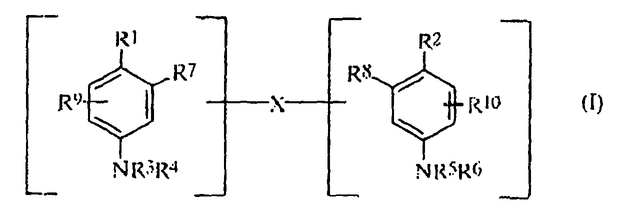
worin
- R¹ und R² siehen unabhängig voneinander für eine Hydroxygruppe oder einen Rest NR'R", wobei R' und R" unabhängig voneinander für Wasserstoff, einen C₁- bis C₄₋Alkylrest, einen C₁- bis C₄-Hydroxyalkylrest und die Verbrückung X stehen und die Gruppe NR'R" auch Bestandteil eines verbrückenden Ringsystems X sein kann,
- die Verbrückung X steht für eine Alkylengruppe mit 2 bis 14 Kohlenstoffatomen, wie beispielsweise eine lineare oder verzweigte Alkylenkette oder einen Alkylenring, die oder der von einer oder mehreren stickstoffhaltigen Gruppen und/oder einem Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- R⁹ und R¹⁰ stehen unabhängig voneinander für ein Wasserstoff- oder Halogenatom, einen C₁- bis C₄-Alkylrest, einen C₁- bis C₄-Monohydroxyalkylrest, einen C₂- bis C₆₋Polyhydroxyalkylrest, einen C₁- bis C₄-Aminoalkylrest oder eine direkte Verbindung zur Verbrückung X,
- R³, R⁴, R⁵, R⁶ R⁷ und R⁸ stehen unabhängig voneinander für ein Wasserstoffatom, eine direkte Bindung zur Verbrückung X oder einen C₁- bis C₄-Alkylrest,
   mit den Maßgaben, dass
- die Verbindungen der Formel (1) nur eine Verbrückung X pro Molekül enthalten und
- die Verbindungen der Formel (l) mindestens eine Aminogruppe enthalten, die mindestens ein Wasserstoffatom trägt,
oder den physiologisch verträglichen Salzen dieser Verbindungen enthält.

Unter keratinischen Fasern werden erfindungsgemäß Pelze, Wolle, Federn und insbesondere menschliche Haare verstanden.

Beispiele für physiologisch verträgliche Salze der erfindungsgemäßen Verbindungen sind die Hydrochloride, Hydrobromide, die Sulfate, die Phosphate, die Acetate, die Propionate, die Citrate und die Lactate.

Beispiele für die als Substituenten in der erfindungsgemäßen Verbindung der Formel (I) genannten C₁- bis C₄-Alkylgruppen sind die Gruppen Methyl, Ethyl, Propyl, Isopropyl und Butyl. Ethyl und Methyl sind bevorzugte Alkylgruppen. Erfindungsgemäß bevorzugte C₁- bis C₄-Alkoxygruppen sind beispielsweise eine Methoxy- oder eine Ethoxygruppe. Weiterhin können als bevorzugte Beispiele für eine C₁- bis C₄₋Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Als bevorzugte Beispiele für C₁- bis C₄-Aminoalkylgruppen gelten erfindungsgemäß Aminomethyl- und 2-Aminoethylgruppen. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab.

In einer ersten bevorzugten Ausführungsform gemäß Formel (I) stehen die Reste R¹ und R² für eine Gruppe NR'R".

Besonders bevorzugt sind die Verbindungen gemäß der allgemeinen Formel (I), bei denen der Rest R' für eine 2-Hydroxyethylgruppe und R" für eine Verbrückung X steht.

Auch erfindungsgemäße Verbindungen gemäß der allgemeinen Formel (I), bei denen die Verbrückung X für die Gruppe -CH₂-CH(OH)-CH₂- steht, sind besonders bevorzugt.

Ebenfalls erfindungsgemäß bevorzugt sind Verbindungen gemäß Formel (I), bei denen die Reste R³ bis R¹⁰ für Wasserstoff stehen.

Als besonders bevorzugtes Beispiel gemäß der ersten Ausführungsform gilt das 1,3-Bis-(N-(2'-hydroxyethyl)-N-(4-aminophenylamino))-2-propanol oder eines seiner physiologisch verträglichen Salze.

In einer dritten bevorzugten Ausführungsform gemäß Formel (I) stehen die Reste R¹ und R² für die Gruppe NR'R", wobei R' und R" ein verbrückendes Ringsystem bilden.

Besonders bevorzugt sind auch solche Verbindungen gemäß Formel (I), bei denen das verbrückende Ringsystem die folgende Struktur aufweist:

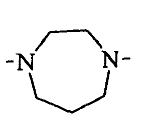

Auch Verbindungen gemäß der allgemeinen Formel (I), bei denen die Reste R³ bis R¹⁰ für ein Wasserstoffatom stehen, sind erfindungsgemäß bevorzugt.

Als besonders bevorzugtes Beispiel gemäß der dritten Ausführungsform gilt das 1,4-Bis-(4-aminophenyl)-diazacycloheptan oder eines seiner physiologisch verträglichen Salze. Es kann auch erfindungsgemäß bevorzugt sein, eine weitere zweikernige Entwicklerkomponente der allgemeinen Formel (I) einzusetzen. Insbesondere bevorzugt sind N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(2-hydroxy-5-aminobenzyl)-piperazin, und N-(4'-Aminophenyl)-p-phenylendiamin sowie ihre physiologisch verträglichen Salze.

Die erfindungsgemäßen farbverändernden Mittel können neben der erfindungsgemäßen Farbstoffkombination mindestens ein weiteres Farbstoffvorprodukt enthalten.

Hinsichtlich der in den erfindungsgemäßen Mitteln einsetzbaren Farbstoffvorprodukte unterliegt die vorliegende Erfindung keinerlei Einschränkungen. Die erfindungsgemäßen Mittel können als Farbstoffvorprodukte
- Oxidationsfarbstoffvorprodukte vom Entwickler- und Kuppler-Typ, und
- Vorstufen naturanaloger Farbstoffe, wie Indol- und Indolin-Derivate,
sowie Mischungen von Vertretern dieser Gruppen enthalten.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen.

Besonders bevorzugt sind p-Phenylendiaminderivate der Formel (II) 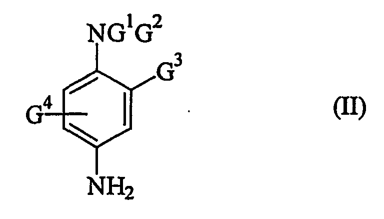 wobei
- G¹ steht für ein Wasserstoffatom, ein C₁- bis C₄-Alkylradikal, ein C₁- bis C₄₋Monohydroxyalkylradikal, ein C₂- bis C₆-Polyhydroxyalkylradikal, ein (C₁- bis C₄)-Alkoxy-(C₁- bis C₄)-alkykadikal, ein 4'-Aminophenylradikal oder ein C₁- bis C₄₋Alkylradikal, das mit einer stickstoffhaltigen Gruppe, einem Phenyl- oder einem 4'-Aminophenylrest substituiert ist;
- G² steht für ein Wasserstoffatom, ein C₁- bis C₄-Alkylradikal, ein C₁- bis C₄₋Monohydroxyalkylradikal, ein C₂- bis C₆-Polyhydroxyalkylradikal, ein (C₁- bis C₄)-Alkoxy-(C₁- bis C₄)-alkylradikal oder ein C₁- bis C₄-Alkylradikal, das mit einer stickstoffhaltigen Gruppe substituiert ist;
- G³ steht für ein Wasserstoffatom, ein Halogenatom, wie ein Chlor-, Brom, Jod- oder Fluoratom, ein C₁- bis C₄-Alkylradikal, ein C₁- bis C₄-Monohydroxyalkylradikal, ein C₂- bis C₄-Polyhydroxyalkylradikal, ein C₁- bis C₄-Hydroxyalkoxyradikal, ein C₁- bis C₄₋Acetylaminoalkoxyradikal, ein C₁- bis C₄- Methylaminoalkoxyradikal oder ein C₁- bis C₄-Carbamoylaminoalkoxyradikal;
- G⁴ steht für ein Wasserstoffatom, ein Halogenatom oder ein C₁- bis C₄-Alkylradikal oder
- wenn G³ und G⁴ in ortho-Stellung zueinander stehen, können sie gemeinsam eine verbrückende α,ω-Alkylendioxogruppe, wie beispielsweise einen Ethylendioxygruppe bilden.

Beispiele für die als Substituenten in den erfindungsgemäßen Verbindungen genannten, C₁- bis C₄-Alkylradikale sind die Gruppen Methyl, Ethyl, Propyl, Isopropyl und Butyl. Ethyl und Methyl sind bevorzugte Alkylradikale. Erfindungsgemäß bevorzugte C₁- bis C₄-Alkoxyradikale sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (II) sind insbesondere die Aminogruppen, C₁- bis C₄₋Monoalkylaminogruppen, C₁- bis C₄-Dialkylaminogruppen, C₁- bis C₄₋Trialkylammoniumgruppen, C₁- bis C₄-Monohydroxyalkylaminogruppen, Imidazolinium und Ammonium.

Besonders bevorzugte p-Phenylendiamine der Formel (II) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-bis-(β-Hydroxyethyl)-p-phenylendiamin, 4-N,N-bis-(β-Hydroxyethyl)amino-2-methylanilin, 4-N,N-bis-(β-Hydroxyethyl)amino-2-chloranilin, 2-(β-Hydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenylp-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (II) sind p-Phenylendiamin, p-Toluylendiamin, 2-(β-Hydroxyethyl)-p-phenylendiamin und. N,N-Bis-(β-hydroxyethyl)-p-phenylendiamin.

Weiterhin kann es erfindungsgemäß bevorzugt sein, als Entwicklerkomponente ein weiteres p-Aminophenolderivat oder eines seiner physiologisch verträglichen Salze einzusetzen.

Besonders bevorzugt sind p-Aminophenolderivate der Formel (III) 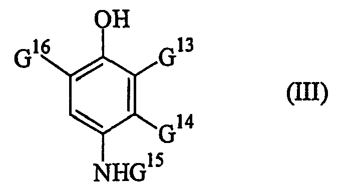 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₆-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₆-Polyhydroxyalkylrest, einen (C₁- bis C₄)-Alkoxy-(C₁- bis C₄)-alkylrest, einen C₁- bis C₄-Aminoalkylrest oder einen C₁- bis C₄₋Cyanoalkylrest,
- G¹⁵ steht für Wasserstoff, einen C₁- bis C₄-Alkylrest, einen C₁- bis C₄₋Monohydroxyalkylrest, einen C₂- bis C₆-Polyhydroxyalkylrest, einen Phenylrest oder einen Benzylrest, und
- G¹⁶ steht für Wasserstoff oder ein Halogenatom.

Die in Formel (III) verwendeten Substituenten sind erfindungsgemäß analog zu den obigen Ausführungen definiert.

Bevorzugte p-Aminophenole der Formel (III) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethylaminomethyl)-phenol sowie ihre physiologisch verträglichen Salze.

Ganz besonders bevorzugte Verbindungen der Formel (III) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)amino-3-amino-pyridin, 2,3-Diamino-6-methoxy-pyridin, 2-(β-Methoxyethyl)amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethyl-amino-4,5,6-triaminopyrimidin, 2,4-Dihydroxy-5,6-diaminopyrimidin und 2,5,6-Tri-aminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorobenzyl)-pyrazol, 4,5-Diamino-1,3-dimethylpyrazol, 4,5-Diamino-3-methyl-1-phenylpyrazol, 4,5-Diamino-l-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2'-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4(β-hydroxyethyl)amino-1-methylpyrazol.

Bevorzugte Pyrazol-Pyrimidin-Derivate sind insbesondere die Derivate des Pyrazol-[1,5-a]-pyrimidin der folgenden Formel (IV) und dessen tautomeren Formen, sofern ein tautomeres Gleichgewicht besteht: 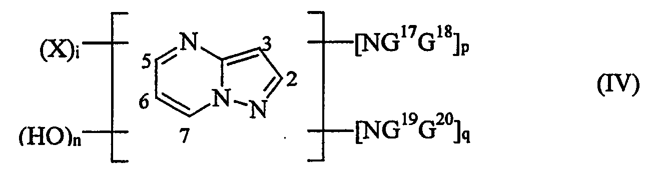 wobei:
- G¹⁷, G¹⁸, G¹⁹ und G²⁰ unabhängig voneinander stehen für ein Wasserstoffatom, ein C₁- bis C₄-Alkylradikal, ein Aryl-Radikal, ein C₁- bis C₄-Hydroxyalkylradikal, ein C₂- bis C₄₋Polyhydroxyalkylradikal ein (C₁- bis C₄)-Alkoxy-(C₁- bis C₄)-alkylradikal, ein C₁- bis C₄₋Aminoalkylradikal, das gegebenenfalls durch ein Acetyl-Ureid- oder Sulfonyl-Radikal geschützt sein kann, ein (C₁- bis C₄)-Alkylamino-(C₁- bis C₄)-alkylradikal, ein Di-[(C₁- bis C₄)-alkyl]-(C₁- bis C₄)-aminoalkylradikal, wobei die Dialkyl-Radikale gegebenenfalls einen Kohlenstoffzyklus oder einen Heterozyklus mit 5 oder 6 Kettengliedern bilden, ein C₁- bis C₄-Hydroxyalkyl- oder ein Di-(C₁- bis C₄)-[Hydroxyalkyl]-(C₁- bis C₄)-aminoalkylradikal,
- die X-Radikale stehen unabhängig voneinander für ein Wasserstoffatom, ein C₁- bis C₄-Alkylradikal, ein Aryl-Radikal, ein C₁- bis C₄-Hydroxyalkyladikal, ein C₂- bis C₄₋Polyhydroxyalkylradikal, ein C₁- bis C₄-Aminoalkylradikal, ein (C₁- bis C₄)-Alkylamino-(C₁- bis C₄)-alkylradikal, ein Di-[(C₁- bis C₄)alkyl]- (C₁- bis C₄)-aminoalkylradikal, wobei die Dialkyl-Radikale gegebenenfalls einen Kohlenstoffzyklus oder einen Heterozyklus mit 5 oder 6 Kettengliedern bilden, ein C₁- bis C₄-Hydroxyalkyl- oder ein Di-(C₁- bis C₄-hydroxyalkyl)-aminoalkylradikal, ein Aminoradikal, ein C₁- bis C₄-Alkyl- oder Di-(C₁- bis C₄-hydroxyalkyl)-aminoradikal, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, daß
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (IV) verwendeten Substituenten sind erfindungsgemäß analog zu den obigen Ausführungen definiert.

Wenn das Pyrazol-[1,5-a]-pyrimidin der obenstehenden Formel (IV) eine Hydroxygruppe an einer der Positionen 2, 5 oder 7 des Ringsystems enthält, besteht ein tautomeres Gleichgewicht, das zum Beispiel im folgenden Schema dargestellt wird:

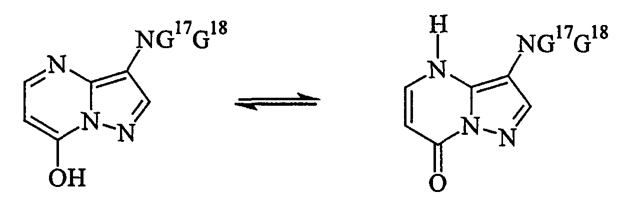

Unter den Pyrazol-[1,5-a]-pyrimidinen der obenstehenden Formel (IV) kann man insbesondere nennen:
- Pyrazol-[1,5-a]-pyrimidin-3,7-diamin;
- 2,5-Dimethyl pyrazol-[1,5-a]-pyrimidin-3,7-diamin;
- Pyrazol-(1,5-a]-pyrimidin-3,5-diamin;
- 2,7-Dimethyl pyrazol-[1,5-a]-pyrimidin-3,5-diamin;
- 3-Amino pyrazol-[1,5-a]-pyrimidin-7-ol;
- 3-Amino pyrazol-[1,5-a]-pyrimidin-5-ol;
- 2-(3-Amino pyrazol-[1,5-a]-pyrimidin-7-ylamino)-ethanol;
- 2-(7-Amino pyrazol-[1,5-a]-pyrimidin-3-ylamino)-ethanol;
- 2-[(3-Amino pyrazol-[1,5-a]-pyrimidin-7-yl)-(2-hydroxy-ethyl)-amino]-ethanol;
- 2-[(7-Amino pyrazol-[1,5-a]-pyrimidin-3-yl)-(2-hydroxy-ethyl)-amino]-ethanol;
- 5,6-Dimethyl pyrazol-[1,5-a]-pyrimidin-3,7-diamin;
- 2,6-Dimethyl pyrazol-[1,5-a]-pyrimidin-3,7-diamin;
- 2,5, N7, N7-Tetramethyl pyrazol-[1,5-a]-pyrimidin-3,7-diamin;
sowie ihre physiologisch verträglichen Salze und ihre tautomeren Formen, wenn ein tautomerisches Gleichgewicht vorhanden ist.

Die Pyrazol-[1,5-a]-pyrimidine der obenstehenden Formel (IV) können wie in der Literatur beschrieben durch Zyklisierung ausgehend von einem Aminopyrazol oder von Hydrazin hergestellt werden.

Erfindungsgemäß ganz besonders bevorzugte Entwicklerkomponenten sind ausgewählt aus p-Phenylendiamin, p-Toluylendiamin, p-Aminophenol, o-Aminophenol, N,N-Bis-(2'-hydroxyethyl)-p-phenylendiamin, 2-(2',5'-Diaminophenoxy)-ethanol, 2-Hydroxymethylamino-4-aminophenol, Bis-(4-aminophenyl)amin, 4-Amino-3-fluorphenol, 4-Amino-2-((diethylamino)-methyl)-phenol, 2-Aminomethyl-4-aminophenol, 2-Hydroxymethyl-4-aminophenol, 4-Amino-2-(2'-hydroxyethoxy)-phenol, Bis-(2-hydroxy-5-aminophenyl)-methan, 2,4,5,6-Tetraaminopyrimidin, 2-Hydroxy-4,5,6-triaminopyrimidin und 4-Hydroxy-2,5,6-triaminopyrimidin, 2,4-Dihydroxy-5,6-diamino-pyrimidin, 2-Dimethylamino-4,5,6-triaminopyrimidin und den Diaminopyrazol-Derivaten nach EP 0 740 931 bzw. WO 94/08970 wie beispielsweise 4,5-Diamino-1-(2'-hydroxyethyl)-pyrazol, 4,5-Diamino-1-(4'-chlorbenzyl)-pyrazol sowie deren physiologisch verträglichen Salzen dieser Verbindungen.

Als Kupplerkomponenten werden in der Regel m-Phenylendiaminderivate, Naphthole, Resorcin und Resorcinderivate, Pyrazolone, Pyridinderivate und m-Aminophenolderivate verwendet.

Erfindungsgemäß bevorzugte Kupplerkomponenten sind:
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 3-Trifluoracetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, 1,3-Dihydroxy-5-(methylamino)-benzol und 3-Ethylamino-4-methylphenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1,3-Bis-(2',4'-diaminophenyl)-propan, 2,6-Bis-(2'-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxy-benzol.

Besonders bevorzugte weitere Kupplerkomponenten sind 1-Naphthol, 1,5-, 2,7- und 1,7-Dihydroxynaphthalin, m-Aminophenol, 5-Amino-2-methylphenol, 5-(2'-Hydroxyethylamino)-3-methylphenol, 2-Chlor-6-methyl-3-aminophenol, 2-Methyl-4-chlor-5-aminophenol, Resorcin, Resorcinmonomethylether, m-Phenylendiamin, 2-Chlorresorcin, 4-Chlorresorcin, 2-Methylresorcin, 5-Methylresorcin, 2,5-Dimethylresorcin, m-Phenylendiamin, 2-Methylamino-3-amino-6-methoxypyridin, 2-Amino-3-hydroxypyridin, 3,4-Dimethyl-pyridin, 2,6-Diydroxy-3,4-dimethylpyridin, 1-Phenyl-3-methyl-pyrazolon-5, 2,4-Diaminophenoxyethanol, 2-Amino-4-(2'hydroxyethyl)aminoanisol 1,3-Bis-(2',4'-diaminophenoxy)-propan, 6-Hydroxyindol sowie deren physiologisch verträglichen Salze.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Die Entwicklerkomponenten sind in den erfindungsgemäßen Mitteln bevorzugt in Mengen von 0,005 bis 20 Gew.-%, vorzugsweise 0,1 bis 5 Gew.-% und die Kupplerkomponenten sind bevorzugt in Mengen von 0,005 bis 20 Gew.-%, vorzugsweise 0,1 bis 5 Gew.%, jeweils bezogen auf das gesamte Mittel, enthalten.

Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe.

Besonders gut als Vorstufen naturanaloger Haarfarbstoffe geeignet sind Derivate des 5,6-Dihydroxyindolins der Formel (V), 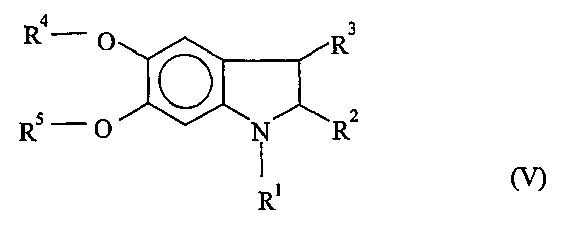 in der unabhängig voneinander
R¹ steht für Wasserstoff, eine C₁-C₄-Alkylgruppe oder eine C₁-C₄-Hydroxy-alkylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der R⁶ steht für eine C₁-C₄-Alkylgruppe, und
R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (VI), 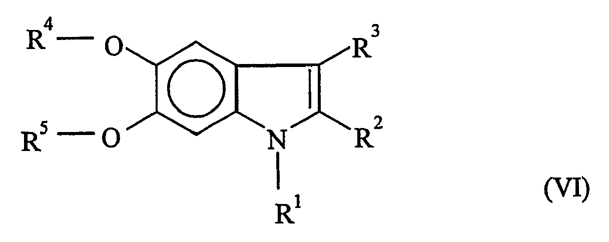 in der unabhängig voneinander
R¹ steht für Wasserstoff, eine C₁-C₄-Alkylgruppe oder eine C₁-C₄-Hydroxyalkylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der R⁶ steht für eine C₁-C₄-Alkylgruppe, und
R⁵ steht für eine der unter R⁴ genannten Gruppen,
sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.%, vorzugsweise 0,2-5 Gew.% enthalten.

Insbesondere bei der Verwendung von Farbstoff-Vorstufen vom Indolin- oder Indol-Typ hat es sich als vorteilhaft erwiesen, als Alkalisierungsmittel eine Aminosäure und/oder ein Oligopeptid einzusetzen.

Neben den Farbstoffvorprodukten können die erfindungsgemäßen Mittel zur weiteren Nuancierung direktziehende Farbstoffe enthalten. Diese sind üblicherweise ausgewählt aus Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, HC Orange 1, Disperse Orange 3, HC Red 1, HC Red 3, HC Red 13, HC Red BN, Basic Red 76, HC Blue 2, HC Blue 12, Disperse Blue 3, Basic Blue 7, Basic Blue 26, Basic Blue 99, HC Violet 1, Disperse Violet 1, Disperse Violet 4, Basic Violet 2, Basic Violet 14, Acid Violet 43, Disperse Black 9, Acid Black 52, Basic Brown 16 und Basic Brown 17 bekannten Verbindungen sowie 1,4-Bis-(β-hydroxyethyl)-amino-2-nitrobenzol, 3-Nitro-4-(β-hydroxyethyl)-aminophenol, 4-Amino-2-nitrodiphenylamin-2'-carbonsäure, 6-Nitro-1,2,3,4-tetrahydrochinoxalin, 2-Hydroxy-1,4-naphthochinon, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Die erfindungsgemäßen Mittel enthalten Farbstoffvorprodukte bevorzugt in einem geeigneten wäßrigen, alkoholischen oder wäßrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, die Farbstoffvorprodukte in eine pulverförmige oder auch Tablettenförmige Formulierung zu integrieren.

Unter wäßrig-alkoholischen Lösungen sind im Sinne der vorliegenden Erfindung wäßrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Die eigentliche oxidative Färbung der Fasern kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Man kann aber die Oxidationsfärbemittel auch zusammen mit einem Katalysator auf das Haar aufbringen, der die Oxidation der Farbstoffvorprodukte, z. B. aktiviert. Solche Katalysatoren sind z. B. Übergangsmetallverbindungen, Iodide, Chinone oder bestimmte Enzyme. Geeignete Enzyme sind z. B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z. B.
- Pyranose-Oxidase und z. B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Das eigentliche Haarfärbemittel wird zweckmäßigerweise unmittelbar vor der Anwendung durch Mischung der Zubereitung des Oxidationsmittels mit der Zubereitung, enthaltend die Farbstoffvorprodukte, hergestellt. Das dabei entstehende gebrauchsfertige Haar-färbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Die erfindungsgemäßen Mittel können weiterhin alle für solche Zubereitungen bekannten Wirk-, Zusatz- und Hilfsstoffe enthalten. In vielen Fällen enthalten diese Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobemsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel

R¹O-(Z)_{x'}

Diese Verbindungen sind durch die folgenden Parameter gekennzeichnet:
Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, daß eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine - COO⁽⁻⁾- oder -SO₃⁽⁻⁾Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl N,N-dimethylammoniumglycinate, beispielsweise das Kokosacylaminopropyldimethyl-ammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethyl-ammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quatemierten Estersalze von Fettsäuren mit 1,2-Dihydroxypropyldialkylaminen. Solche Produkte werden beispielsweise unter den Warenzeichen Stepantex® , Dehyquart® und Armocare® vertrieben. Die Produkte Armocare® VGH-70, ein N,N-Bis(2-Palmitoyloxyethyl)dimethylammonium-chlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®⁻Quat 3270 und 3272 (Hersteller: Th. Goldschmidt; diquaternäre Polydimethylsiloxane, Quaternium-80).
Ein Beispiel für ein als kationisches Tensid einsetzbares quaternäres Zuckerderivat stellt das Handelsprodukt Glucquat® 100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, - hydroxide oder -alkoholate als Katalysatoren verwendet werden. Die Verwendung von Produkten mit eingeengter Homologenverteilung kann bevorzugt sein.

Weiterhin können die erfindungsgemäßen Mittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.

Bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR® 400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat® 100 (Poly(dimethyldiallylammoniumchlorid)), Merquat® 550 (Dimethyldiallylammoniumchlorid-Acrylamid-Copolymer) und Merquat® 280 (Dimethyldiallylammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat® 734 und Gafquat® 755 im Handel erhältlich.
- Vinylpyrrolidon-Methoimidazoliniumchlorid-Copolymere, wie sie unter der Bezeichnung Luviquat® angeboten werden.
- quaternierter Polyvinylalkohol
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquaternium-2, Polyquaternium-10 und Polyquaternium-22.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Coming; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil® -Quat 3270 und 3272 (Hersteller: Th. Goldschmidt; diquaternäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyltrimethylammoniumchlorid/Acrylat-Copolymere und Octylacrylamid/Methylmethacrylat/tert-Butylaminoethylmethacrylat/2- Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein zweiter Gegenstand der Erfindung ist ein Verfahren zur Färbung von Keratinfasern, bei dem die erfindungsgemäßen Mittel auf die zu färbenden Fasern aufgebracht und nach einer Einwirkungszeit wieder ausgespült oder mit einem Shampoo ausgewaschen wird.

Ein dritter Gegenstand der Erfindung ist die Verwendung eines erfindungsgemäßen Mittels zur Färbung keratinischer Fasern.

Die folgenden Beispiele sollen den Gegenstand der vorliegenden Anmeldung verdeutlichen.

### Ausführungsbeispiele

### 1. Zusammensetzung der Färbecreme

| Teilmischung A | |
|---|---|
| Hydrenol® D¹ | 8,5 g |
| Lorol® techn.² | 2,0 g |
| Eumulgin® B2³ | 0,75 g |
| Texapon® NSO⁴ | 20,0 g |
| Dehyton® K⁵ | 12,5 g |
| Destilliertes Wasser | 30,0 g |

| | |
|---|---|
| 1 C₁₆₋₁₈-Fettalkohol (INCI-Bezeichnung: Cetearyl alcohol) (Cognis) | |
| ² C₁₂₋₁₈-Fettalkohol (INCI-Bezeichnung: Coconut alcohol) (Cognis) | |
| ³ Cetylstearylalkohol mit ca. 20 Mol EO (INCI-Bezeichnung: Ceteareth-20) (Cognis) | |
| ⁴ Natriumlaurylethersulfat (ca. 28% Aktivsubstanz; INCI-Bezeichnung: Sodium Laureth Sulfate) (Cognis) | |
| ⁵ Fettsäureamid-Derivat mit Betainstruktur der Formel R-CONH(CH₂)₃N⁺(CH₃)₂CH₂COO⁻ (ca. 30 % Aktivsubstanz; INCI-Bezeichnung: Cocoamidopropyl Betaine) (Cognis) | |

Die Substanzen Hydrenol® D, Lorol® und Eumulgin® B2 wurden bei 80°C aufgeschmolzen, mit dem 80°C heißen Wasser, enthaltend Texapon® NSO und Dehyton® K, vermischt und unter starkem Rühren emulgiert. Danach wurde die Emulsion unter schwachem Rühren abgekühlt.

| Teilmischung B | |
|---|---|
| Natriumsulfit | 1,0 g |
| Ammoniumsulfat | 1,0 g |
| Farbstoffvorprodukte | 2,5 mmol (jeweils) |
| Ammoniak (25%ige Lösung) | ad pH 10,0 |
| Wasser | 10,0 g |

Die Farbstoffvorprodukte wurden in dem 50°C heißen Wasser unter Zugabe von Natriumsulfit, Ammoniumsulfat und Ammoniak gelöst. Anschließend wurde die Farbstoffvorproduktlösung (Teilmischung B) zur Emulsion (Teilmischung A) gegeben, mit Ammoniak auf pH10 eingestellt und mit Wasser auf 100g aufgefüllt. Es wurde bis zum Erreichen der Raumtemperatur weitergerührt.

### 2. Färbung der Keratinfasern

Die so erhaltene Färbecreme wurde im Verhältnis 1:1 mit Wasser, einer 1%igen wässrigen H₂O₂-Lösung beziehungsweise einer 9%igen wässrigen H₂O₂-Lösung vermischt und auf die Strähnen standardisierten, zu 80% ergrauten, aber nicht besonders vorbehandelten Menschenhaares (Fa. Klugmann) aufgetragen. Nach 30-minütiger Einwirkungszeit bei 32°C wurde das Haar gespült, mit einem üblichen Haarwaschmittel ausgewaschen und anschließend getrocknet.

Die Ergebnisse der Färbeversuche sind der folgenden Tabelle zu entnehmen, in der die folgenden Kuppler- und Entwicklerkomponenten verwendet wurden:

### Kupplerkomponente:

K: 2,4-Dichlor-3-aminophenol-hydrochlorid

### Entwicklerkomponenten:

E1: 1,3-Bis-(N-(2'-hydroxyethyl)-N-(4-aminophenylamino))-2-propanol-tetrahydrochlorid -hydrat
E2: 1,10-Bis-(2',5'-diaminophenyl)-1,4,7,10-tetraoxadecan-tetrahydrochlorid
E3: 1,4-Bis-(4'-aminophenyl)-diazacycloheptan-tetrahydrochlorid-hydrat
E4: 1-(2'Hydroxyethyl)-4,5-diaminopyrazolsulfat
E5: 4-Hydroxy-2,5,6-triaminopyrimidinsulfat
E6: 2,4,5,6-Tetraaminopyrimidinsulfat
E7: 1-(2'-Hydroxyethyl)-2,5-diaminobenzolsulfat

| **Farbstoff-Kombination** | **Luftoxidation** | **Farbergebnis** | |
|---|---|---|---|
| | | **1% H**_{**2**}**O**_{**2**} | **9% H**_{**2**}**O**_{**2**} |
| K + E1 | 20F4 | 20 F 8 | 20 F 8 |
| | dunkeltürkis | schwarzblau | schwarzblau |
| K + E1 + E4 | 9D5 | 12 F 8 | 13 F 8 |
| | bois-de rose | portweinrot | dunkelmagenta |
| K + E2 + E7 | 24 F 4 | 20 F 8 | 20 F 8 |
| | dunkeltürkis | schwarzblau | schwarzblau |
| K + E3 + E4 | 8F5 | 13F8 | 13F8 |
| | dunkelbraun | dunkelmagenta | dunkelmagenta |
| K + E1 + E5 | 9 E 3 | 18F5 | 19F4 |
| | graubraun | dunkelviolett | schwarzblau |
| K + E1 + E6 | 21 F3 | 20 F 4 | 20 F 4 |
| | gewitterblau | tintenblau | tintenblau |
| K + E1 + E7 | 24 F 4 | 20 F 8 | 20 F 8 |
| | dunkeltürkis | schwarzblau | schwarzblau |

## Patentansprüche

1. Mittel zum Färben von Keratinfasern, **dadurch gekennzeichnet, dass** es in einem zum Färben geeigneten Medium als Oxidationsfarbstoffvorprodukte eine Kombination aus
a) 2,4-Dichlor-3-aminophenol und
b) mindestens einer Verbindung gemäß Formel (I) 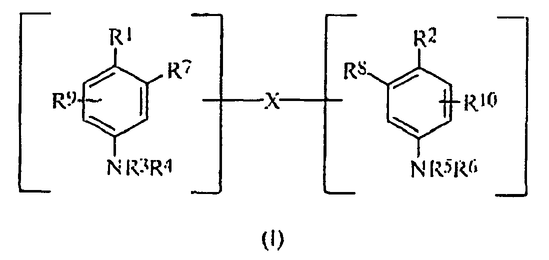
worin
- R¹ und R² stehen unabhängig voneinander für eine Hydroxygruppe oder einen Rest NR'R", wobei R' und R" unabhängig voneinander für Wasserstoff, einen C₁- bis C₄₋Alkylrest, einen C₁- bis C₄-Hydroxyalkylrest und die Verbrückung X stehen und die Gruppe NR'R" auch Bestandteil eines verbrückenden Ringsystems X sein kann,
- die Verbrückung X steht für eine Alkylengruppe mit 2 bis 14 Kohlenstoffatomen, wie beispielsweise eine lineare oder verzweigte Alkylenkette oder einen Alkylenring, die oder der von einer oder mehreren stickstoffhaltigen Gruppen und/oder einem Schwefel- oder Stickstoffatomen unterbrochen oder beendet sein kann und eventuell durch einen oder mehrere Hydroxyl- oder C₁- bis C₈₋Alkoxyreste substituiert sein kann, oder eine direkte Bindung,
- R⁹ und R¹⁰ stehen unabhängig voneinander für ein Wasserstoff- oder Halogenatom, einen C₁- bis C₄-Alkylrest, einen C₁- bis C₄-Monohydroxyalkylrest, einen C₂- bis C₆₋Polyhydroxyalkylrest, einen C₁- bis C₄-Aminoalkylrest oder eine direkte Verbindung zur Verbrückung X,
- R³, R⁴, R⁵, R⁶ R⁷ und R⁸ stehen unabhängig voneinander für ein Wasserstoffatom, eine direkte Bindung zur Verbrückung X oder einen C₁- bis C₄-Alkylrest,
mit den Maßgaben, dass
- die Verbindungen der Formel (I) nur eine Verbrückung X pro Molekül enthalten und
- die Verbindungen der Formel (I) mindestens eine Aminogruppe enthalten, die mindestens ein Wasserstoffatom trägt,
oder den physiologisch verträglichen Salzen dieser Verbindungen enthält.

2. Mittel gemäß Anspruch 1, **dadurch gekennzeichnet, dass** die Reste R¹ und R² für eine Gruppe NR'R" stehen.

3. Mittel gemäß Anspruch 2, **dadurch gekennzeichnet, dass** R' für eine 2-Hydroxyethylgruppe und R" für eine Verbrückung X steht.

4. Mittel gemäß einem der Ansprüche 1 bis 3, **dadurch gekennzeichnet, dass** die Verbrückung X für die Gruppe -CH₂-CH(OH)-CH₂- steht.

5. Mittel gemäß einem der Ansprüche 1 bis 4, **dadurch gekennzeichnet, dass** die Reste R³ bis R¹⁰ für Wasserstoff stehen.

6. Mittel gemäß einem der Ansprüche 1 bis 5, **dadurch gekennzeichnet, dass** die Verbindung gemäß Formel (I) 1,3-Bis-(N-(2'-hydroxyethyl)-N-(4-aminophenylamino))-2-propanol oder eines seiner physiologisch verträglichen Salze ist.

7. Mittel gemäß Anspruch 1, **dadurch gekennzeichnet, dass** die Reste R¹ und R² für einen Rest NR'R" stehen, wobei R' und R" ein verbrückendes Ringsystems bilden.

8. Mittel nach Anspruch 7, **dadurch gekennzeichnet, dass** das verbrückende Ringsystem die folgende Struktur aufweist:

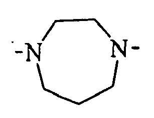

9. Mittel gemäß einem der Ansprüche 7 oder 8, **dadurch gekennzeichnet, dass** die Reste R³ bis R¹⁰ jeweils für ein Wasserstoffatom stehen.

10. Mittel gemäß einem der Ansprüche 7 bis 9, **dadurch gekennzeichnet, dass** die Verbindung gemäß Formel (I) 1,4-Bis-(4-aminophenyl)-diazacycloheptan oder eines seiner physiologisch verträglichen Salze ist.

11. Mittel nach einem der Ansprüche 1 bis 10, **dadurch gekennzeichnet, dass** es mindestens noch eine weitere Entwicklerkomponente enthält.

12. Mittel nach Anspruch 11, **dadurch gekennzeichnet, dass** die weitere Entwicklerkomponente ausgewählt ist aus p-Phenylendiamin, p-Toluylendiamin, p-Aminophenol, o-Aminophenol, N,N-Bis-(2'-hydroxyethyl)-p-phenylendiamin, 2-(2',5'-Diaminophenoxy)-ethanol, 2-Hydroxymethylamino-4-aminophenol, Bis-(4-aminophenyl)amin, 4-Amino-3-fluorphenol, 4-Amino-2-((diethylamino)-methyl)-phenol, 2-Aminomethyl-4-aminophenol, 2-Hydroxymethyl-4-aminophenol, 4-Amino-2-(2'-hydroxyethoxy)-phenol, Bis-(2-hydroxy-5-aminophenyl)-methan, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diamino-pyrimidin, 2-Dimethylamino-4,5,6-triaminopyrimidin 4,5-Diamino-1-(2'-hydroxyethyl)-pyrazol und 4,5-Diamino-1-(4'-chlorbenzyl)pyrazol sowie deren physiologisch verträglichen Salzen.

13. Mittel nach einem der Ansprüche 1 bis 12, **dadurch gekennzeichnet, dass** es mindestens eine weitere Kupplerkomponente enthält.

14. Mittel nach Anspruch 13, **dadurch gekennzeichnet, dass** die Kupplerkomponente ausgewählt ist aus 1-Naphthol, 1,5-, 2,7- und 1,7-Dihydroxynaphthalin, m-Aminophenol, 5-Amino-2-methylphenol, 5-(2'-Hydroxyethylamino)-3-methylphenol, 2-Chlor-6-methyl-3-aminophenol, 2-Methyl-4-chlor-5-aminophenol, Resorcin, Resorcinmonomethylether, m-Phenylendiamin, 2-Chlorresorcin, 4-Chlorresorcin, 2-Methylresorcin, 5-Methylresorcin, 2,5-Dimethylresorcin, m-Phenylendiamin, 2-Methylamino-3-amino-6-methoxypyridin, 2-Amino-3-hydroxypyridin, 2,6-Dihydroxy-3,4-Dimethylpyridin, 1-Phenyl-3-methyl-pyrazolon-5, 2,4-Diaminophenoxyehanol, 2-Amino-4-(2'-hydroxyethyl)amino-anisol, 1,3-Bis-(2',4'-diaminophenoxy)-propan, 6-Hydroxyindol sowie deren physiologisch verträglichen Salzen.

15. Mittel nach einem der Ansprüche 1 bis 14, **dadurch gekennzeichnet, dass** es weiterhin Vorstufen naturanaloger Farbstoffe, wie Indol- und Indolin-Derivate, enthält.

16. Mittel nach einem der Ansprüche 1 bis 15, **dadurch gekennzeichnet, dass** es als Vorstufen naturanaloger Farbstoffe 5,6-Dihydroxyindol, 5,6-Dihydroxyindolin enthält.

17. Mittel nach einem der Ansprüche 1 bis 16, **dadurch gekennzeichnet, dass** Entwicklerkomponeten in einer Menge von 0,005 bis 20 Gew.-%, vorzugsweise 0.1 bis 5 Gew.-%, und Kupplerkomponenten in einer Menge von 0,005 bis 20 Gew.-%, vorzugsweise 0,1 bis 5 Gew.-%, jeweils bezogen auf das gesamte Oxidationsfärbemittel, enthalten sind.

18. Mittel nach einem der Ansprüche 1 bis 17, **dadurch gekennzeichnet, dass** weiterhin mindestens ein direktziehender Farbstoff enthalten ist.

19. Verfahren zum Färben von Keratinfasern, **dadurch gekennzeichnet, dass** ein Mittel zum Färben von Keratinfasern nach einem der Ansprüche 1 bis 18 auf die zu färbenden Fasern aufgebracht und nach einer Einwirkungszeit wieder ausgespült oder mit einem Shampoo ausgewaschen wird.

20. Verwendung eines Mittels gemäß den Ansprüchen 1 bis 18 zur Färbung keratinischer Fasern.

## Claims

1. Agent for dyeing keratin fibres, **characterized in that** it comprises, in a medium suitable for dyeing, as oxidation dye precursors, a combination of
a) 2,4-dichloro-3-aminophenol and
b) at least one compound according to formula (I) 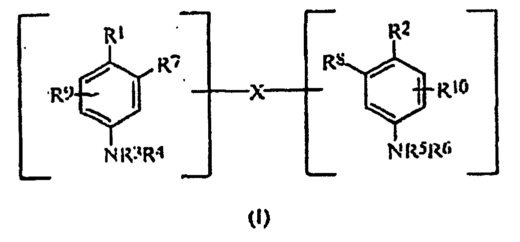
in which
- R¹ and R², independently of one another, are a hydroxy group or a radical NR'R", where R' and R", independently of one another, are hydrogen, a C₁- to C₄-alkyl radical, a C₁- to C₄-hydroxyalkyl radical and the bridge X, and the group NR'R" may also be a constituent of a bridging ring system X,
- the bridge X is an alkylene group having 2 to 14 carbon atoms, such as, for example, a linear or branched alkylene chain or an alkylene ring which may be terminated or interrupted by one or more nitrogen-containing groups and/or a sulphur or nitrogen atom and may possibly be substituted by one or more hydroxyl or C₁- to C₈-alkoxy radicals, or a direct bond,
- R⁹ and R¹⁰, independently of one another, are a hydrogen atom or halogen atom, a C₁- to C₄-alkyl radical, a C₁- to C₄-monohydroxyalkyl radical, a C₂- to C₆-polyhydroxyalkyl radical, a C₁- to C₄₋aminoalkyl radical or a direct bond to the bridge X,
- R³, R⁴, R⁵, R⁶, R⁷ and R⁸, independently of one another, are a hydrogen atom, a direct bond to the bridge X or a C₁- to C₄-alkyl radical,
with the provisos that
- the compounds of the formula (I) comprise only one bridge X per molecule and
- the compounds of the formula (I) comprise at least one amino group which carries at least one hydrogen atom,
or the physiologically compatible salts of these compounds.

2. Agent according to Claim 1, **characterized in that** the radicals R¹ and R² are a group NR'R''.

3. Agent according to Claim 2, **characterized in that** R' is a 2-hydroxyethyl group and R" is a bridge X.

4. Agent according to one of Claims 1 to 3, **characterized in that** the bridge X is the group -CH₂-CH(OH)-CH₂.

5. Agent according to one of Claims 1 to 4, **characterized in that** the radicals R³ to R¹⁰ are hydrogen.

6. Agent according to one of Claims 1 to 5, **characterized in that** the compound according to formula (I) is 1,3-bis(N-(2'-hydroxyethyl)-N-(9-aminophenylamino))-2-propanol or one of its physiologically compatible salts.

7. Agent according to Claim 1, **characterized in that** the radicals R¹ and R² are a radical NR'R", where R' and R" form a bridging ring system.

8. Agent according to Claim 7, **characterized in that** the bridging ring system has the following structure:

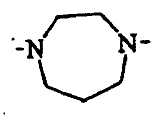

9. Agent according to one of Claims 7 and 8, **characterized in that** the radicals R³ to R¹⁰ are in each case a hydrogen atom.

10. Agent according to one of Claims 7 to 9, **characterized in that** the compound according to formula (I) is 1,4-bis(4-aminophenyl)diazacycloheptane or one of its physiologically compatible salts.

11. Agent according to one of Claims 1 to 10, **characterized in that** it at least also comprises a further developer component.

12. Agent according to Claim 11, **characterized in that** the further developer component is chosen from p-phenylenediamine, p-tolylenediamine, p-aminophenol, o-aminophenol, N,N-bis (2'-hydroxyethyl) -p-phenylenediamine, 2-(2',5'-diaminophenoxy)ethanol, 2-hydroxy-methylamino-4-aminophenol, bis (4-aminophenyl) amine, 4-amino-3-fluorophenol, 4-amino-2-((diethylamino)-methyl)phenol, 2-aminomethyl-4-aminophenol, 2-hydroxymethyl-4-aminophenol, 4-amino-2-(2'-hydroxyethoxy)-phenol, bis(2-hydroxy-5-aminophenyl)methane, 2,4,5,6-tetraaminopyrimidine, 2-hydroxy-4,5,6-triaminopyrimidine, 4-hydroxy-2,5,6-triaminopyrimidine, 2,4-di-hydroxy-5,6-diaminopyrimidine, 2-dimethylamino-4,5,6-triaminopyrimidine, 4,5-diamino-1-(2'-hydroxyethyl)-pyrazole and 4,5-diamino-1-(4'-chlorobenzyl)pyrazole and physiologically compatible salts thereof.

13. Agent according to one of Claims 1 to 12, **characterized in that** it comprises at least one further coupler.component.

14. Agent according to Claim 13, **characterized in that** the coupler component is chosen from 1-napththol, 1,5-, 2,7- and 1,7-dihydroxynaphthalene, m-aminophenol, 5-amino-2-methylphenol, 5-(2'-hydroxyethylamino)-3-methylphenol, 2-chloro-6-methyl-3-aminophenol, 2-methyl-4-chloro-5-aminophenol, resorcinol, resorcinol monomethyl ether, m-phenylenediamine, 2-chlororesorcinol, 4-chlororesorcinol, . 2-methylresorcinol, 5-methylresorcinol, 2,5-dimethylresorcinol, m-phenylenediamine, 2-methylamino-3-amino-6-methoxypyridine, 2-amino-3-hydroxypyridine, 2,6-dihydroxy-3,4-dimethylpyridine, 1-phenyl-3-methylpyrazolone-5, 2,4-diaminophenoxyethanol, 2-amino-4-(2'-hydroxyethyl)-aminoanisole, 1,3-bis(2',4'-diaminophenoxy)propane, 6-hydroxyindole and physiologically compatible salts thereof.

15. Agent according to one of Claims 1 to 14, **characterized in that** it further comprises precursors of nature-analogous dyes, such as indole and indoline derivatives.

16. Agent according to one of Claims 1 to 15, **characterized in that** it comprises 5,6-dihydroxyindole, 5,6-dihydroxyindoline as precursors of nature-analogous dyes.

17. Agent according to one of Claims 1 to 16, **characterized in that** developer components are present in an amount of from 0.005 to 20% by weight, preferably 0.1 to 5% by weight, and coupler components are present in an amount of from 0.005 to 20% by weight, preferably 0.1 to 5% by weight, in each case based on the total oxidation colorant.

18. Agent according to one of Claims 1 to 17, **characterized in that** at least one direct dye is furthermore present.

19. Method of dyeing keratin fibres, **characterized in that** an agent for dyeing keratin fibres according to one of Claims 1 to 18 is applied to the fibres to be dyed and, after a contact time, is rinsed out again or washed out with a shampoo.

20. Use of an agent according to Claims 1 to 18 for dyeing keratin fibres.

## Revendications

1. Agent de coloration de fibres kératiniques, **caractérisé en ce que** dans un milieu approprié pour la coloration, il contient, en tant que précurseur de colorant d'oxydation, une combinaison comprenant
a) du 2,4-dichloro-3-aminophénol et
b) au moins un composé de formule (I) 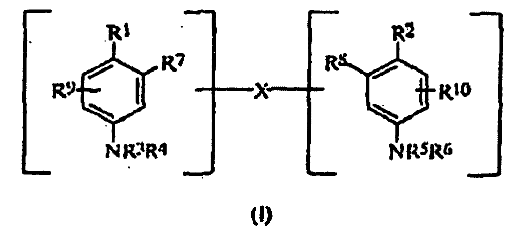
dans laquelle
- R¹ et R² représentent chacun indépendamment l'un de l'autrè un groupe hydroxy ou un groupe NR'R", où R' et R" représentent chacun indépendamment l'un de l'autre un atome d'hydrogène, un groupe alkyle en C₁-C₄, un groupe hydroxy(alkyle en C₁-C₄) et le groupe de pontage X, et le groupe NR'R" peut être un constituant d'un système cyclique de pontage X,
- le pontage X représente un groupe alkylène comprenant 2 à 14 atomes de carbone comme, par exemple, une chaîne alkylène linéaire ou ramifiée ou un cycle alkylène, qui peut être interrompu(e) ou terminé(e) par un ou plusieurs groupes azotés et/ou un atome de soufre ou d'azote et le cas échéant, peut être substitué(e) par un ou plusieurs groupes hydroxy ou alcoxy en C₁-C₃, ou une liaison directe,
- R⁹ et R¹⁰ représentent chacun indépendamment l'un de l'autre un atome d'hydrogène ou d'halogène, un groupe alkyle en C₁-C₄, monohydroxy(alkyle en C₁-C₄), polyhydroxy(alkyle en C₂-C₆), amino(alkyle en C₁-C₄), ou une liaison directe avec le groupe de pontage X,
- R³, R⁴, R⁵, R⁶, R⁷ et R⁸ représentent chacun indépendamment l'un de l'autre un atome d'hydrogène, une liaison directe avec le groupe de pontage X, ou un groupe alkyle en C₁-C₄,
sous réserve que
- les composés de formule (I) ne contiennent qu'un groupe de pontage X par molécule et
- les composés de formule (I) contiennent au moins un groupe amino qui porte au moins un atome d'hydrogène,
ou un de ses sels physiologiquement acceptables.

2. Agent selon la revendication 1, **caractérisé en ce que** les groupes R¹ et R² représentent un groupe NR'R".

3. Agent selon la revendication 2, **caractérisé en ce que** R' représente un groupe 2-hydroxyéthyle et R" un groupe de pontage X.

4. Agent selon l'une quelconque des revendications 1 à 3, **caractérisé en ce que** le pontage X représente un groupe -CH₂-CH(OH)-CH₂-.

5. Agent selon l'une quelconque des revendications 1 à 4, **caractérisé en ce que** les groupes R³ à R¹⁰ représentent un atome d'hydrogène.

6. Agent selon l'une quelconque des revendications 1 à 5, **caractérisé en ce que** le composé de formule (I) est le 1,3-bis-(N-(2'-hydroxyéthyl)-N-(4-aminophénylamino))-2-propanol ou un de ses sels physiologiquement acceptables.

7. Agent selon la revendication 1, **caractérisé en ce que** les groupes R¹ et R² représentent un groupe NR'R", R' et R" formant un système cyclique de pontage.

8. Agent selon la revendication 7, **caractérisé en ce que** le système cyclique de pontage présente la structure suivante :

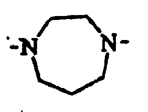

9. Agent selon la revendication 7 ou 8, **caractérisé en ce que** les groupes R³ à R¹⁰ représentent chacun un atome d'hydrogène.

10. Agent selon l'une quelconque des revendications 7 à 9, **caractérisé en ce que** le composé de formule (I) est le 1,4-bis-(4-aminophényl)-diazacycloheptane ou un de ses sels physiologiquement acceptables.

11. Agent selon l'une quelconque des revendications 1 à 10, **caractérisé en ce qu'**il contient au moins un autre composant développeur.

12. Agent selon la revendication 11, **caractérisé en ce que** l'autre composant développeur est choisi parmi la p-phénylènediamine, la p-toluylène-diamine, le p-aminophénol, l'o-aminophénol, la N,N-bis-(2'-hydroxyéthyl)-p-phénylènediamine, le 2-(2',5'-diaminophénoxy)-éthanol, le 2-hydroxyméthylamino-4-aminophénol, la bis-(4-aminophényl)amine, le 4-amino-3-fluorophénol, le 4-amino-2-((diéthylamino)-méthyl)-phénol, le 2-aminométhyl-4-aminophénol, le 2-hydroxyméthyl-4-aminophénol, le 4-amino-2-(2'-hydroxyéthoxy)-phénol, le bis-(2-hydroxy-5-aminophényl)-méthane, la 2,4,5,6-tétra-aminopyrimidine, la 2-hydroxy-4,5,6-triaminopyrimidine, la 4-hydroxy-2,5,6-triaminopyrimidine, la 2,4-dihydroxy-5,6-diaminopyrimidine, la 2-diméthylamino-4,5,6-triaminopyrimidine, le 4,5-diamino-1-(2'-hydroxyéthyl)-pyrazole et le 4,5-diamino-1-(4'-chlorobenzyl)pyrazole, ainsi que leurs sels physiologiquement acceptables.

13. Agent selon l'une quelconque des revendications 1 à 12, **caractérisé en ce qu'**il contient au moins un autre composant de couplage.

14. Agent selon la revendication 13, **caractérisé en ce que** le composant de couplage est choisi parmi le 1-naphtol, les 1,5-, 2,7- et 1,7-dihydroxynaphtalènes, le m-aminophénol, le 5-amino-2-méthylphénol, le 5-(2'-hydroxyéthylamino)-3-méthylphénol, le 2-chloro-6-méthyl-3-aminophénol, le 2-méthyl-4-chloro-5-aminophénol, le résorcinol, le résorcinol-monométhyléther, la m-phénylènediamine, le 2-chlororésorcinol, le 4-chlororésorcinol, le 2-méthylrésorcinol, le 5-méthylrésorcinol, le 2,5-diméthylrésorcinol, la m-phénylènediamine, la 2-méthylamino-3-amino-6-méthoxypyridine, la 2-amino-3-hydroxypyridine, la 2,6-dihydroxy-3,4-diméthylpyridine, la 1-phényl-3-méthyl-pyrazolone-5, le 2,4-diaminophénoxyéthanol, le 2-amino-4-(2'-hydroxyéthyl)amino-anisol, le 1,3-bis-(2',4'-diaminophénoxy)-propane, le 6-hydroxyindole ainsi que leurs sels physiologiquement acceptables.

15. Agent selon l'une quelconque des revendications 1 à 14, **caractérisé en ce qu'**il contient en outre des précurseurs de colorants naturels, tels que les dérivés d'indole et d'indoline.

16. Agent selon l'une quelconque des revendications 1 à 15, **caractérisé en ce qu'**il contient, en tant que précurseur de colorants naturels, le 5,6-dihydroxyindole ou la 5,6-dihydroxyindoline.

17. Agent selon l'une quelconque des revendications 1 à 16, **caractérisé en ce qu'**il contient des composants développeurs dans une proportion de 0,005 à 20 % en poids, de préférence de 0,1 à 5 % en poids et des composants de couplage dans une proportion de 0,005 à 20 % en poids, de préférence de 0,1 à 5 % en poids, toujours par rapport au colorant d'oxydation total.

18. Agent selon l'une quelconque des revendications 1 à 17, **caractérisé en ce qu'**il contient en outre au moins un colorant montant directement sur la fibre.

19. Procédé de coloration de fibres kératiniques, **caractérisé en ce que** l'on applique sur les fibres à colorer un agent de coloration de fibres kératiniques selon l'une quelconque des revendications 1 à 18, et on l'élimine après un certain temps d'action par rinçage ou par lavage avec un shampooing.

20. Utilisation d'un agent selon les revendications 1 à 18 pour la coloration de fibres kératiniques.
